# EUROPEAN PATENT SPECIFICATION

(11) **EP 1 490 333 B1**
(45) Date of publication and mention of the grant of the patent: **20.01.2010**
(21) Application number: 03730380.7
(22) Date of filing: 27.03.2003
(51) Int. Cl.: C07D 207/34, A61K 31/40, A61P 3/06

(54) **NEW ATORVASTATIN SALTS AND PHARMACEUTICAL COMPOSITIONS CONTAINING THEM**
NEUE ATORVASTATINSALZE UND PHARMAZEUTISCHE ZUSAMMENSETZUNGEN, DIE DIESE ENTHALTEN
NOUVEAUX SELS D'ATORVASTATINE ET COMPOSITIONS PHARMACEUTIQUES LES CONTENANT

(30) Priority: 28.03.2002 HU 0201083
(43) Date of publication of application: 29.12.2004
(73) Proprietor: Richter Gedeon Nyrt., 1103 Budapest (HU)
(72) Inventor: FISCHER, János, H-1014 Budapest (HU); VUKICS, Krisztina, H-1111 Budapest (HU); ERDELYI, Péter, H-1103 Budapest (HU); HEGEDÜS, Béla, H-1113 Budapest (HU); TIHANYI, Károly, H-1117 Budapest (HU); VASTAG, Mónika, H-1134 Budapest (HU); LEVAI, Sándor, H-2051 Biatorbágy (HU); BALINT, Sándorné, H-1112 Budapest (HU); LANCZOS, Krisztina, H-1031 Budapest (HU)
(74) Representative: Bublak, Wolfgang
(86) International application number: PCT/HU2003/000024
(87) International publication number: WO 2003/082816

(56) References cited:
- WO-A-01/76566
- WO-A-97/03959

## Description

The invention relates to salts of atorvastatin formed with lysine, arginine and ornithine. The invention also relates to amorphous, as well as polymorph modifications of these salts and pharmaceutical compositions with cholesterol level decreasing activity, containing the above compounds as active ingredients.

Atorvastatin (its chemical name: (3*R*,5*R*)-7-[3-phenyl-4-[(phenylamino)carbonyl]-2-(4-fluorophenyl)-5-(1-methyl-ethyl)-pyrrol-1-yl]-3,5-dihydroxy-heptanoic acid) is known as cholesterol level decreasing compound. A few pharmaceutically acceptable salts of atorvastatin are described in the European Patent Number of EP 409 281. In the claims of the above patent the following salts are included: mono-sodium salt, mono-potassium salt, hemi-calcium salt, N-methyl-glucamine salt, hemi-magnesium salt, hemi-zinc salt, 1-deoxy-1-methylamino-D-glucitol salt. The following bases are listed in the description as salt forming components: sodium hydroxide, potassium hydroxide, lithium hydroxide, calcium hydroxide, 1-deoxy-2-methylamino-D-glucitol, magnesium hydroxide, zinc hydroxide, aluminium hydroxide, iron(II) and iron(III) hydroxide, ammonium hydroxide and organic bases, as for example N-methyl-glucamine, choline, arginine and the like.

The active ingredient of atorvastatin containing drug, which is on the market since 1997, is the hemi-calcium salt of atorvastatin, obviously the most suitable for pharmaceutical application from the salts described in the patent above. Said patent does not contain any observation for possible polymorphism of the compound.

In the patent Number of WO 97/03959 three different crystal modifications of atorvastatin hemi-calcium salt are already described, called crystal forms I, II and IV, at the same time the product of the above patent is characterized as an amorphous material, the filtration property and the industrial producibility of which are not satisfactory.

In the description the form I is emphasized from the crystalline atorvastatin hemi-calcium salts, which occurs as trihydrate. According to the description the synthesis of this modification is carried out by crystallization from aqueous alcoholic solution using seeding crystal or adding tert-butyl-methyl ether to the solution.

The advantage of the crystalline atorvastatin hemi-calcium salt is, that the product is more easily filterable compered to the amorphous product: while the filtration of the amorphous form takes more than one hour, the filtration of the same amount of the crystalline product takes only a few seconds.

The patent Number of WO 97/03958 describes the crystal form III of atorvastatin hemi-calcium salt, which can be obtained from the crystal form II by a quite complicated method. Finally according to the patent Number of WO 01/36384 the crystal form V of atorvastatin hemi-calcium salt is also known, which is obtained under similar conditions as the crystal form I.

The different publications in the literature represent - surprisingly - considerably different opinion about the amorphous modification of atorvastatin hemi-calcium salt. One of the reasons of the contradiction is, that the amorphous modification is poorly filterable and this results in technological problems. The other reason of the contradiction is the biological observation, according to which the absorption of the amorphous modification of atorvastatin hemi-calcium salt is worse, than that of the crystalline form. Oishi and coworkers /Yakuri to Chiryo, 26 (8), 1241-1252 (1998)/ found, that in human the crystalline form of atorvastatin hemi-calcium salt is absorbed to significantly better rate and larger extent, than the amorphous form of atorvastatin hemi-calcium salt. The amorphous form of atorvastatin hemi-calcium salt is described in the following patents Number of WO 97/03960, WO 00/71116, WO 01/28999 and WO 01/42209.

It is known, that the atorvastatin hemi-calcium salt is cholesterol level decreasing agent, the activity of which is based on the inhibition of HMG-CoA reductase enzyme. However its absorption is not perfect, only about 30 % of the applied dose is absorbed. See Corsini D. M. and coworker: Pharm.Res. 14 (11) Suppl. S253 (1997). The systemic bioavailability is only 12 %.

The adequate concentration of drugs in living organisms, that is the adequate bioavailability is essential for their therapeutic effect. The bioavailability is basically determined by two factors: the absorption and the metabolic stability. The rate and extent of absorption of an active ingredient of a drug can significantly be influenced by its different forms: salts, polymorph modifications or solvates.

The selection of the most appropriate salt and crystal form is particularly important, when the bioavailability of the drug is limited by the absorption. The use of a certain salt or crystal form and/or solvate provides such intrinsic features to the dosage forms, which can not be achieved by simple technological solutions, such as grinding or other drug formulation methods (adding auxiliaries, disintegrants and the like).

The in vivo absorption can be examined by Caco-2 test, by which the drug penetration is measured through human intestinal epithelial cell-layer (Literature: Pade V. et al: J.Pharm.Sci. 87, 1604-7 /1998/ and Artursson P. et al: Bichem.Biophys.Res.Comm., 175, 880-885 /1991/). The essence of the method is, that microtablets are pressed from the drug substances to be examined without adding auxiliaries. The microtablets are made under the same conditions with the same pressing force, therefor the rate of penetration through cell-layer is determined by the intrinsic features of the examined substances.

According to our experiments the salts of atorvastatin formed with basic amino acids according to our invention are penetrated significantly better, than the crystal form I of atorvastatin hemi-calcium salt.

Therefor our invention is based on the observation, that the salts of atorvastatin formed with lysine, arginine, or ornithine, which can be obtained under properly choosen conditions show more advantageous properties than the known ones.

According to the above mentioned facts the invention relates to the salts of atorvastatin formed with a basic amino acid of the general formula (I), where the meaning of R is a group originated from lysine, arginine or ornithine, and the amorphous or polymorph modifications thereof, thus the salts of atorvastatin formed with L-, D- and DL-lysine, L-arginine and L-ornithine.

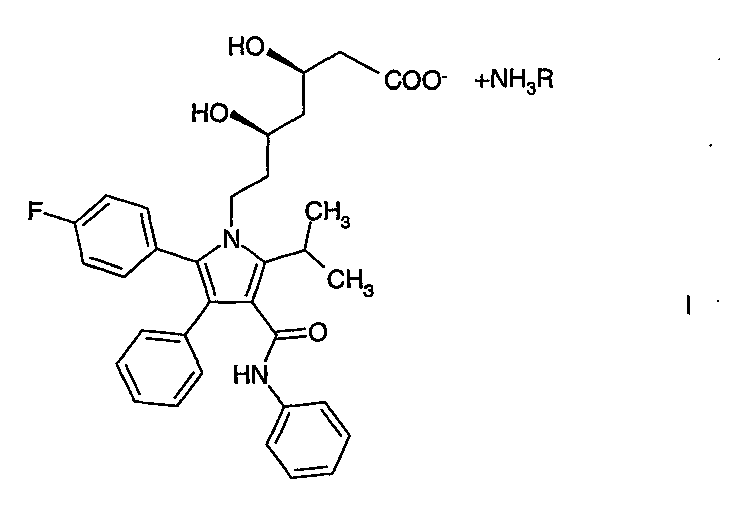

The invention also relates to polymorph modification "A" of the salt of atorvastatin formed with L-lysine, where the X-ray powder-diffraction interlayer distances of the molecule are the following: 9,351; 5,126; **4,693;** 4,558; 4,239; 4,055; 3,828; 3,617; 3,441; 3,343; 3,203; 3,107; 2,918; 2,709; 2,595 (Å), the characteristic infrared bands are: 3643, 3322, 1646, 1583, 1423, 1084, 775, 699 cm⁻¹, the Raman bands are: 2981, 1647, 1480, 734, 677, 635, 513, 200 cm⁻¹, as well as the "B" polymorph modification of the salt of atorvastatin formed with L-lysine, where the X-ray powder-diffraction interlayer distances of the molecule are the following: 20,971; 13,143; 8,606; 7,512; 6,687; 5,168; 4,778; **4,559;** 4,131; 3;941; 3,839; 3,734; 3,639; 3,236; 3,025; 2,793(Å), the characteristic infrared bands are: 3370, 1652, 1412, 1317, 1214, 922, 692, 550 cm⁻¹, the Raman bands are: 3066, 2950, 1650, 1528, 924, 475, 232, 215 cm⁻¹.

The invention relates to polymorph modification "C" of the salt of atorvastatin formed with L-lysine, where the X-ray powder-diffraction interlayer distances of the molecule are the following: 18,686; 13,075; 9,177; 7,762; 6,753, 5,250; **4,873;** 4,725; 4,528; 4,211; 3,923; 3,706; 3,455; 3,135 és 3,038 (Å), the characteristic infrared bands are: 3365, 1649, 1596, 1527, 1217, 1031, 851, 877, 810, 747, 582, 505 cm⁻¹, the Raman bands are: 3067, 2914, 1646, 1605, 1529, 1371, 1160, 1001, 826 cm⁻¹, as well as the amorphous modification of the salt of atorvastatin formed with L-lysine, where the characteristic infrared bands are: 1648, 1596, 1559, 1530, 1220, 916, 886, 808, 749, 732, 690, 616, 507 cm⁻¹, the Raman bands are: 3062, 2922, 1648, 1604, 1530, 1481, 1244, 1159, 999, 820 cm⁻¹.

Furthermore, the invention relates to the process for the synthesis of salts of atorvastatin formed with a basic amino acid of the general formula (I), where the meaning of R is a group originated from lysine, arginine or ornithine, and the polymorph modifications thereof, the following way: the protecting groups of an atorvastatin derivative, preferably a compound of the formula (II) are cleaved in a solvent, the so obtained atorvastatin acid is reacted with a proper amino acid or the salt thereof in a solvent, the desired polymorph modification is crystallized if necessary with the change of solvents.

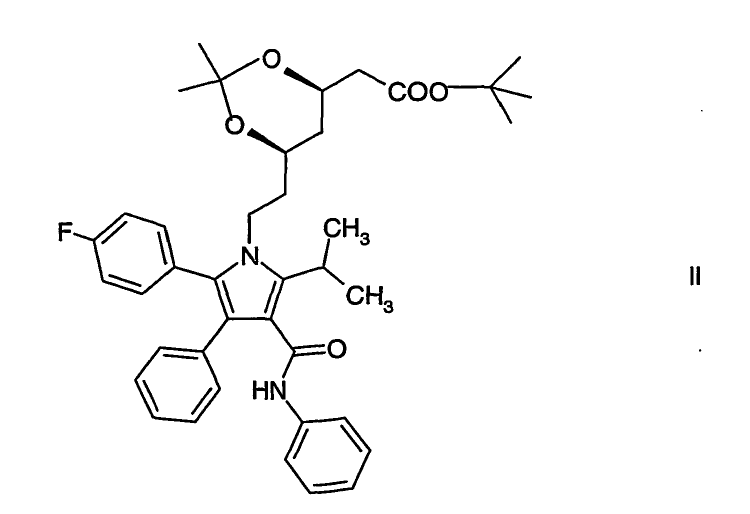

The invention also relates to pharmaceutical compositions containing the salts of atorvastatin formed with a basic amino acid of the general formula (I), where the meaning of R is a group originated from lysine, arginine or ornithine, and the polymorph modifications thereof as active ingredient in an amount of 0.10-99.90 w/w % and known carriers and auxiliaries in an amount of 0.10-99.90 w/w %, which have cholesterol level decreasing activity.

Surprisingly it was found, that the new atorvastatin salts of the invention show better penetration properties, than the crystal form I of atorvastatin hemicalcium salt. This property is not due to the difference of the specific surface of the certain salts, since for example the specific surface of atorvastatin L-lysine salt (polymorph "B", see later) is 1.1 m²/g, while the specific surface of crystal I of atorvastatin hemi-calcium salt is 8.0 m²/g. Therefor on the basis of specific surfaces the atorvastatin L-lysine salt should have had the lower penetration.

From among the atorvastatin salts of our invention formed with basic amino acids the atorvastatin L-lysine salt has - according to our experiments - three reproducible crystalline form, which are called crystal modifications "A", "B" and "C", and it has an amorphous modification as well.

During the study of the formation of the different modifications we found, that the crystal modification "A" of atorvastatin L-lysine salt is formed, when ethyl acetate is used as crystallization solvent, the crystal modification "B" of atorvastatin L-lysine salt is formed, when aqueous ethanol is used as crystallization solvent, while the crystal modification "C" of atorvastatin L-lysine salt is formed, when aqueous isopropanol is used as crystallization solvent. The amorphous atorvastatin L-lysine salt can be obtained, when dichloromethane is used for the salt formation and crystallization.

The crystal modifications of atorvastatin L-lysine salts obtained according to the above procedures are new substances and were studied by spectroscopic methods generally used in the area of polymorphy. The obtained characteristic spectroscopic data are shown below for each crystal modification.

The X-ray diffraction study of different polymorph modifications of atorvastatin L-lysine salt was carried out by Philips PW 1840 powder diffractometer using the following parameters:

| | |
|---|---|
| CuK_{α} radiation: | 30 kV, 30 mA |
| Goniometer speed: | 0.05 ⁰2θ/s |
| Sensitivity: | 2 x 10³ cps |
| T. C.: | 5 seconds |
| Gap width: | 0.05 mm |

The different modifications of atorvastatin L-lysine salt can be characterized by their IR and Raman spectroscopic data as well. The infrared spectra of the modifications were recorded by Perkin-Elmer Spectrum 1000 type FT-IR instrument in KBr pastilles using 4 cm⁻¹ spectral resolution. In the table those bands are given, the presence of which in the infrared spectra of the sample proves the said crystal modification.

The FT-Raman spectra of the modifications were recorded by Perkin-Elmer Spectrum 2000 type instrument using 4 cm⁻¹ spectral resolution, 300 mW irradiation energy obtained by Nd:YAG laser and using 30 fold spectrum accumulation.

Characteristic spectroscopic data of modification "A":
**X-ray powder diffraction interlayer distances:** 9,351; 5,126; **4,693;** 4,558; 4,239; 4,055; 3,828; 3,617; 3,441; 3,343; 3,203; 3,107; 2,918; 2,709; 2,595 (Å).
**IR:** 3643, 3322, 1646, 1583, 1423, 1084, 775, 699 cm⁻¹
**Raman:** 2981, 1647, 1480, 734, 677, 635, 513, 200 cm⁻¹

Characteristic spectroscopic data of modification "B":
**X-ray powder diffraction interlayer distances:** 20,971; 13,143; 8,606; 7,512; 6,687; 5,168; 4,778; **4,559;** 4,131; 3,941; 3,839; 3,734; 3,639; 3,236; 3,025; 2,793(Å).
**IR:** 3370, 1652, 1412, 1317, 1214, 922, 692, 550 cm⁻¹
**Raman:** 3066, 2950, 1650, 1528, 924, 475, 232, 215 cm⁻¹

Characteristic spectroscopic data of modification "C":
**X-ray powder diffraction interlayer distances:** 18,686; 13,075; 9,177; 7,762; 6,753, 5,250;
**4,873;** 4,725; 4,528; 4,211; 3,923; 3,706; 3,455; 3,135 és 3,038 (Å).
**IR:** 3365, 1649, 1596, 1527, 1217, 1031, 851, 877, 810, 747, 582, 505 cm⁻¹
**Raman:** 3067, 2914, 1646, 1605, 1529, 1371, 1160, 1001, 826 cm⁻¹

Characteristic spectroscopic data of amorphous atorvastatin L-lysine salt:
**IR:** 1648, 1596, 1559,1530, 1220, 916, 886, 808, 749, 732, 690, 616, 507 cm⁻¹
**Raman:** 3062, 2922, 1648, 1604, 1530, 1481, 1244, 1159, 999, 820 cm⁻¹
The invention is illustrated in details by the following examples:

### Example 1

### Modification "A" of atorvastatin L-lysine salt

36 ml of 10 w/w % aqueous hydrochloric acid is added to a solution of 10.0 g (15.3 mmol) of protected atorvastatin /its chemical name: 1,1-dimethylethyl (4*R*-*cis*)-6-[2-[3-phenyl-4-[(phenylamino)carbonyl]-2-(4-fluorophenyl)-5-(1-methylethyl)-1*H-*pyrrol-1-yl]ethyl]-2,2-dimethyl-1,3-dioxane-4-acetate; see K. L. Baumann et al.: Tetrahedron Letters, 33, 2283-2284 (1992)/ in 100 ml of tetrahydrofuran in a 250 ml round bottom flask. The so obtained mixture is stirred at 25 °C for 8 h, then 8.8 ml of 50 w/w % aqueous sodium hydroxide is added dropwise and stirring is continued for further 8 h. After completion of the reaction the phases are separated, the organic layer is washed with 65 ml of aqueous sodium chloride solution, and the solvent is evaporated in vacuum. 100 ml of water and 65 ml of ethyl acetate are added to the residue. The pH of the stirred emulsion is adjusted to 4.0 by adding 10 w/w % aqueous orthophosphoric acid solution. The phases are separated and the organic layer, which contains the atorvastatin acid, is washed with 40 ml of aqueous sodium chloride solution twice, dried over sodium sulfate and filtered. The filtrate is diluted with ethyl acetate in a 100 ml volumetric flask exactly to 100 ml and the concentration of the solution is determined by titrimetry (14.5 mmol atorvastatin acid in 100 ml solution). 2.38 g (14.5 mmol) of L-lysine monohydrate is added to the solution of atorvastatin acid in ethyl acetate and the obtained suspension is stirred at 25 °C for 24 h. The crystalline product is filtered off and washed with 30 ml of ethyl acetate twice to yield 10.21 g (95 %) of the title compound. The characteristic bands of its infrared spectrum are the following: 3643, 3322, 1646, 1583, 1423, 1084, 775, 699 cm⁻¹, the characteristic bands of its Raman spectrum are as follows: 2981, 1647, 1480, 734, 677, 635, 513, 200 cm⁻¹. Mp.: 141 °C (decomp.)

### Example 2

### Modification "A" of atorvastatin L-lysine salt

1.76 g (2.5 mmol) of atorvastatin L-lysine salt obtained according to Example 1 is suspended in 12 ml of a mixture of acetone:water = 94:6. The suspension is boiled to obtain a clear solution and 6 ml of acetone is added to the warm solution. The stirred solution is cooled and stirred at 0 °C for 5 h. The precipitated crystalline product is filtered off, washed with 4 ml of a mixture of acetone:water = 96:4 twice, and dried in a vacuum desiccator to yield 1.56 g (89 %) of the title compound as white crystal. Mp.: 141 °C (decomp.)

### Example 3

### Modification "B" of atorvastatin L-lysine salt

9.21 g (13.1 mmol) of atorvastatin L-lysine salt obtained according to Example 1 is suspended in 184 ml of ethanol. The suspension is boiled and water (13.4 ml) is added dropwise to the warm stirred suspension to obtain clear solution. The stirred solution is cooled and stirred at 0 °C for 5 h. The precipitated crystalline product is filtered off, washed with 30 ml of a mixture of ethanol:water = 94:6 twice, and dried in a vacuum desiccator to yield 8.01 g (87 %) of the title compound as white crystal. The characteristic bands of its infrared spectrum are the following: 3370, 1652, 1412, 1317, 1214, 922, 692, 550 cm⁻¹, the characteristic bands of its Raman spectrum are as follows: 3066, 2950, 1650, 1528, 924, 475, 232, 215 cm⁻¹. Mp.: 152 °C (decomp.)

### Example 4

### Modification "C" of atorvastatin L-lysine salt

9.21 g (13.1 mmol) of atorvastatin L-lysine salt obtained according to Example 1 is suspended in 184 ml of isopropanol. The suspension is boiled and water (14 ml) is added dropwise to the warm stirred suspension to obtain clear solution. The stirred solution is cooled and stirred at 0 °C for 5 h. The precipitated crystalline product is filtered off, washed with 30 ml of a mixture of isopropanol:water = 93:7 twice, and dried in a vacuum desiccator to yield 8.29 g (90 %) of the title compound as white crystal. The characteristic bands of its infrared spectrum are the following: 3365, 1649, 1596, 1527, 1217, 1031, 851, 877, 810, 747, 582, 505 cm⁻¹, the characteristic bands of its Raman spectrum are as follows: 3067, 2914, 1646, 1605, 1529, 1371, 1160, 1001, 826 cm⁻¹. Mp.: 142 °C (decomp.)

### Example 5

### Amorphous atorvastatin L-lysine salt

7.1 ml of 10 w/w % aqueous hydrochloric acid is added to a solution of 1.96 g (3 mmol) of protected atorvastatin in 40 ml of tetrahydrofuran in a 100 ml round bottom flask. The so obtained mixture is stirred at 25 °C for 8 h, then 1.74 ml of 50 w/w % aqueous sodium hydroxide is added dropwise and stirring is continued for further 8 h. After completion of the reaction the phases are separated, the organic layer is washed with 15 ml of aqueous sodium chloride solution, and the solvent is evaporated in vacuum. 60 ml of water and 60 ml of dichloromethane are added to the residue. The pH of the stirred emulsion is adjusted to 4.0 by adding 10 w/w % aqueous orthophosphoric acid solution. The phases are separated and the organic layer, which contains the atorvastatin acid, is washed with 20 ml of aqueous sodium chloride solution twice, dried over sodium sulfate and filtered. The filtrate is diluted with dichloromethane in a 100 ml volumetric flask exactly to 100 ml and the concentration of the solution is determined by titrimetry (2.67 mmol atorvastatin acid in 100 ml solution). 0.39 g (2.67 mmol) of L-lysine monohydrate is added to the solution of atorvastatin acid in dichloromethane and the obtained suspension is stirred at 25 °C for 24 h. The crystalline product is filtered off and washed with 10 ml of dichloromethane twice to yield 1.53 g (72 %) of the title compound. The characteristic bands of its infrared spectrum are the following: 1648, 1596, 1559, 1530, 1220, 916, 886, 808, 749, 732, 690, 616, 507 cm⁻¹, the characteristic bands of its Raman spectrum are as follows: 3062, 2922, 1648, 1604, 1530, 1481, 1244, 1159, 999, 820 cm⁻¹.

### Example 6

### Atorvastatin DL-lysine salt

25 ml of 0.136 mmol/l atorvastatin solution in ethyl acetate is prepared according to the method described in Example 1, and 497 mg (3.4 mmol) of DL-lysine is added. The obtained suspension is evaporated to dryness. The residue is dissolved by boiling in 55 ml of a mixture of ethanol:water = 96:4. After cooling the solution the precipitated crystals are filtered off, washed with 3 ml of a cold mixture of ethanol:water = 96:4 twice, and dried in vacuum desiccator at room temperatur to yield 1.55 g (65%) of the title compound. The characteristic bands of its infrared spectrum are the following: 3370, 1651, 1596, 1558, 1509, 1436, 1316, 1215, 1158, 841, 748, 692 cm⁻¹. Mp.: 145 °C (decomp.)

### Example 7

### Atorvastatin D-lysine salt

520 mg (3.56 mmol) of D-lysine is added to 31.2 ml of 114 mmol/dm³ atorvastatin solution in ethyl acetate. The suspension is stirred at room temperature for 20 h, the precipitated atorvastatin D-lysine salt is filtered off and recrystallized from 18.5 ml of a mixture of ethanol:water = 94:6 (the dissolution is achieved by addition of 0.9 ml of water). The product is dried in vacuum desiccator to yield 1.89 g (76 %) of the title compound. The characteristic bands of its infrared spectrum are the following: 3356, 1651, 1596, 1559, 1529, 1510, 1437, 1316, 1158, 840, 693 cm⁻¹.

### Example 8

### Atorvastatin L-arginine salt

To a solution of 1.29 g (2.32 mmol) of atorvastatin in 96.5 ml of ethyl acetate, prepared according to the method described in Example 1, a solution of 0.39 g (2.24 mmol) of L-arginine in 50 ml of methanol is added. The mixture is stirred at 25 °C for 1 h, then the solvents are evaporated in vacuum. 20 ml of diisopropyl ether is added to the residue and the so obtained suspension is stirred at 25 °C for 5 h. The precipitated product is filtered off and washed with 5 ml of diisopropyl ether twice to yield 1.57 g (96 %) of the title compound. The characteristic bands of its infrared spectrum are the following: 2963, 1596, 1528, 1509, 1437, 1401, 1313, 1223, 1157, 843, 753, 692 cm⁻¹. Mp.: 138 °C (decomp.)

### Example 9

### Atorvastatin L-ornithine salt

To a stirred solution of 1.65 g (2.84 mmol) of atorvastatin in 66 ml of ethyl acetate, prepared according to the method described in Example 1, 0.37 g (2.84 mmol) of L-ornithine is added. The solvent is evaporated. The residue is suspended in 50 ml of ethanol, boiled and 0.7 ml of water is added. The slightly opalesque warm solution is filtered, then the stirred filtrate is cooled and stirred at 0 °C for 5 h. The precipitated product is filtered off, washed with 5 ml of a mixture of ethanol:water = 99:1 twice and dried in vacuum desiccator to yield 0.92 g (47 %) of the title compound. The characteristic bands of its infrared spectrum are the following: 3376, 1654, 1596, 1510, 1436, 1316, 1214, 1158, 842, 746, 690 cm⁻¹. Mp.: 150 °C.

### Example 10

### Experiments with Caco-2 test

Tested compounds: polymorph modification "B" of atorvastatin L-lysine salt atorvastatin L-arginine salt
   atorvastatin L-ornithine salt
   polymorph modification I of atorvastatin hemi-calcium salt
Reference compounds: [¹⁴C] mannitol (NEN^{™}, USA)
   corticosterone (Sigma-Aldrich, Hungary)
   sulfasalazine (Sigma-Aldrich, Hungary)
Cell-line: Caco-2 human epithelial cells (HTB-37; ATCC, USA)

### Applied method:

The absorption of the compounds was studied in Caco-2 *in vitro* cell-culture model (See: Pade V., Stavchansky S.: Link between drug absorption solubility and permeability measurements in Caco-2 cells. J. Pharm. Sci., 87, 1604-7, 1998; and Artursson P., Karlsson J.: Correlation between oral drug absorption in humans and apparent drug permeability coefficients in human intestinal epithelial (Caco-2) cells. Biochem. Biophys. Res.Commun. 175, 880-885, 1991).

In the model the absorption of atorvastatin through human epithelial (Caco-2) mono cell-layer was measured in such a way, that the microtablets prepared from the test compounds were placed on the mono cell-layers in the vessels containing physiological saline, then the time dependent amount of the test substance, which passed through the mono cell-layer was determined from the saline solution of the other side of the mono cell-layer by chromatography.

The microtablets were prepared prior to the experiments from the test substances; thus 30 mg of polymorph "B" of atorvastatin L-lysine salt, 30 mg of polymorph I of atorvastatin hemi-calcium salt, 30 mg of atorvastatin L-arginine salt or 30 mg of atorvastatin L-ornithine salt was pressed for 30 seconds at a pressure of 1 Kbar.

During the experiments 4 µmol base equivalent amounts were placed on the mono cell-layers from the microtablets.

We used transcellularly (through cells) and paracellularly (in cells) absorbed reference compounds, the absorption values of which determined in similar system is known from the literature, to validate and prove the reproducibility of our method. (See: Artursson P., Karlsson J.: Correlation between oral drug absorption in humans and apparent drug permeability coefficients in human intestinal epithelial (Caco-2) cells. Biochem. Biophys. Res.Commun. 175, 880-885, 1991; and Liang-Shang L.Gan, Yanni S, Thakker D.R.: Modulation of the tight junctions of the Caco-2 cell monolayers by H2 antagonists. Pharm. Res., 15, 53-57 1998).

During the experiments the reference compounds were placed on the mono cell-layer (luminal side, which *in vivo* equals the inner side of the intestine) in solution, the amount of the test substance, which passed through the mono cell-layer (abluminal side, which *in vivo* equals the blood circulation) was determined by chromatography, then the absorption coefficient characteristic for the absorption of the compounds was calculated (the English abbreviation used in the literature: Papp value). The so obtained Papp values of the reference compounds are in good accordance with the data published in the literature.

At the end of the absorption experiments the intactness of the epithelial mono cell-layers was microscopically checked after tainting with toluidine blue. The amount of the test and reference compounds in the samples was determined by HPLC.

### Discussion of the results:

In our experiments we compared the absorption of atorvastatin from the different salts of the invention formed with L-lysine, L-ornithine and L-arginine, then we compared the absorption of atorvastatin from polymorph "B" modification of L-lysine salt selected from the above three salts of the invention and from the polymorph I of the known hemi-calcium salt in Caco-2 absorption model.

The different modifications of atorvastatin salts were used as microtablets in situ.

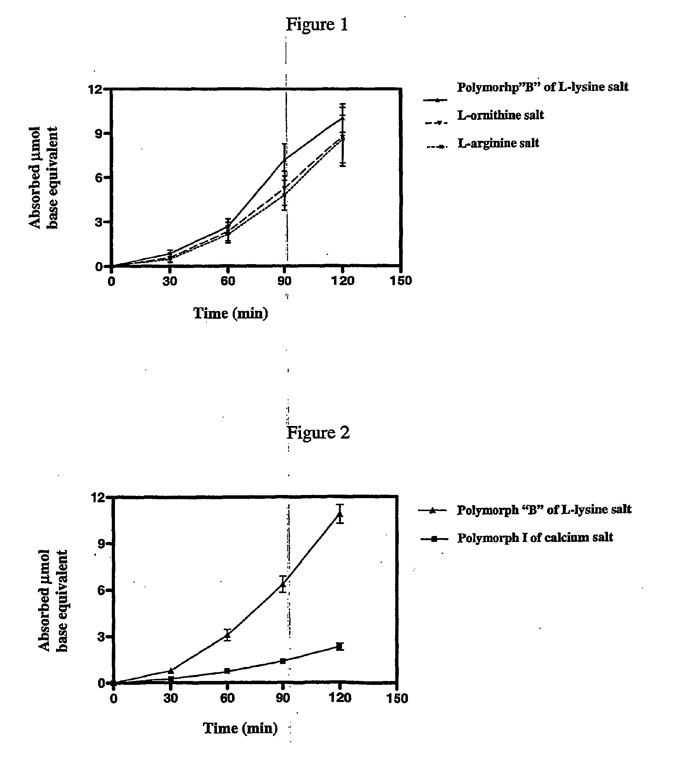

According to our results there was no difference in the absorption of atorvastatin, when the microtablets prepared from the three basic salts, L-lysine, L-ornithine or L-arginine salts, were placed on the mono layers (see: Figure 1).

When the microtablets prepared from the selected polymorph "B" modification of L-lysine salt of the invention were placed on the mono cell-layer, the appearance of atorvastatin on the abluminal side of the mono cell-layers was significantly faster, than that of microtablets prepared from polymorph I of hemi-calcium salt (see:Figure 2).

Therefor our results show that in Caco-2 model the absorption of atorvastatin from polymorph "B" modification of L-lysine salt of the invention is significantly better, than from polymorph I of hemi-calcium salt.

The bioavailability of the marketed hemi-calcium salt of atorvastatin is low, about 12 % (See: Gibson D.M., Stem R.H., Abel R.B., Whitfield L.R.: Absolute bioavailability of atorvastatin in man. Pharm.Res. 14 (11), Suppl. S253, 1997). The two main reasons of this are on one hand the considerable "first pass" (before entering the systemic circulation) metabolism of atorvastatin, on the other hand the inadequate absorption of it. A considerable amount of the administered hemi-calcium salt of atorvastatin does not absorb during passing the intestines.

Consequently according to our discovery backed by our measurements the new, well-absorbed salts of our invention can be the active ingredients of an atorvastatin composition with better bioavailability, than the known one.

### Example 11

### Solid pharmaceutical compositions containing atorvastatin L-lysine salt

The compositions contain an amount of modification "B" of L-lysine salt equal to 10-20 mg of atorvastatin acid as tablets, coated tablets or granules, which can be filled into capsules.

The active ingredient is homogenized with pharmaceutically acceptable one or more diluting agents, such as lactose monohydrate, cellulose derivatives, such as microcrystalline cellulose, methyl cellulose, hydroxypropyl cellulose, carboxymethyl cellulose sodium, as well as starch, pregelatinized starch, inorganic salts, such as CaCO₃, CaHPO₄, waxes, and sugar alcohols.

Auxiliaries generally used in pharmaceutical industry for dry or wet granulation are applied as binding agents, such as starch, cellulose derivatives or polyvinyl pyrrolidone. Stearic acid, Ca or Mg stearate can be used as lubricants. Disintegrating additives can be the following: microcrystalline cellulose, starch glycolate sodium, sodium alginate and the like. Pharmacetucally acceptable colouring agents, flavouring agents and other known auxiliaries can also be used in the compositions.

### Example A

### Preparation of tablets

| Component | Amount |
|---|---|
| Atorvastatin L-lysine salt "B" | 12.6 g |
| Ca carbonate | 39.5 g |
| Lactose monohydrate | 22.5 g |
| Microcrystalline cellulose | 53.5 g |
| Carboxymethyl cellulose Na | 9.8 g |
| Hydroxypropyl cellulose | 9.6 g |
| Mg stearate | 2.5 g |
| Total | 150.0 g |

The above components are pressed to tablets after proper pretreatment. Each of the so obtained 1000 tablets contain an amount of L-lysine salt equal to 10 mg of atorvastatin acid.

### Example B

### Preparation of coated tablets

| Component | Amount |
|---|---|
| Atorvastatin L-lysine salt "B" | 25.2 g |
| Pregelatinized starch | 72.5 g |
| Lactose monohydrate | 42.0g |
| Microcrystalline cellulose | 116.5 g |
| Starch glycolate Na | 19.6 g |
| Hydroxypropyl cellulose | 19.2 g |
| Mg stearate | 5.0 g |
| Total | 300.0 g |

Coated tablets are made by using the following coating mixture from the tablets prepared according to the usual method applied in pharmaceutical industry.

| | |
|---|---|
| Colloidal silicon dioxide | 0.045 g |
| Titanium dioxide | 0.244 g |
| Lactose monohydrate | 0.390 g |
| Macrogol 6000 | 0.394 g |
| Hydroxypropyl methylcellulose | 3.925 g |
| Colouring agent | 0.002 g |
| Total: | 5.000 g |

Each of the so obtained 1000 coated tablets contain an amount of L-lysine salt equal to 20 mg of atorvastatin acid.

### Example C

### Preparation of capsules

| Component | Amount |
|---|---|
| Atorvastatin L-lysin salt "B" | 12.6 g |
| Lactose monohydrate | 22.5 g |
| Microcrystalline cellulose | 63.4 g |
| Mg stearate | 1.5 g |
| Total | 100.0 g |

After homogenization the ingredients are filled into hard gelatin capsules, each of the capsules contain an amount of L-lysine salt equal to 10 mg of atorvastatin acid.

## Claims

1. The compounds of the general formula (I) - wherein the meaning of R is a group formula (II), (III) or (IV) - and the amorphous or polymorph modifications thereof.

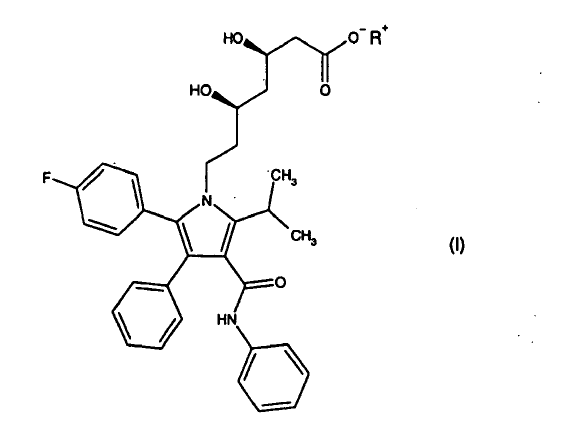

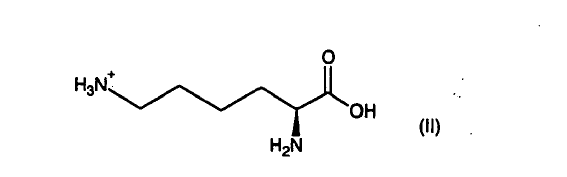

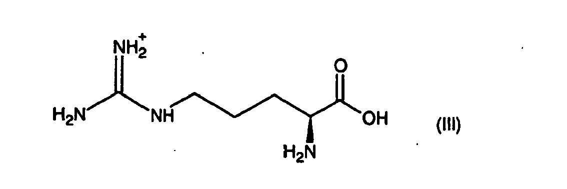

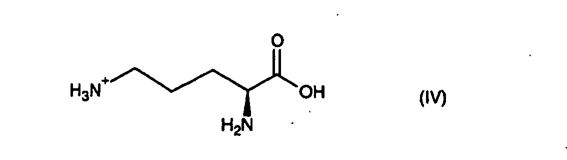

2. A compound according to claim 1, which is Atorvastatin L-, D- and DL-lysine salts.

3. A compound according to claim 1, which is Atorvastatin L-lysine salt.

4. A compound according to claim 1, which is Atorvastatin L-arginine salt.

5. A compound according to claim 1, which is Atorvastatin L-ornithine salt.

6. The polymorph modification "A" of atorvastatin L-lysine salt of claim 3, **characterized by** the following X-ray powder-diffraction interlayer distances of the molecule: 9,351; 5,126; **4,693;** 4,558; 4,239; 4,055; 3,828; 3,617; 3,441; 3,343; 3,203; 3,107; 2,918; 2,709; 2,595 (Å), the characteristic infrared bands are: 3643, 3322, 1646, 1583, 1423, 1084, 775, 699 cm⁻¹, the Raman bands are: 2981, 1647, 1480, 734, 677, 635, 513, 200 cm⁻¹.

7. The polymorph modification "B" of atorvastatin L-lysine salt of claim 3, **characterized by** the following X-ray powder-diffraction interlayer distances of the molecule: 20,971; 13,143; 8,606; 7,512; 6,687; 5,168; 4,778; **4,559;** 4,131; 3,941; 3,839; 3,734; 3,639; 3,236; 3,025; 2,793(Å), the characteristic infrared bands are: 3370, 1652, 1412, 1317, 1214, 922, 692, 550 cm⁻¹, the Raman bands are: 3066, 2950, 1650, 1528, 924, 475, 232, 215 cm⁻¹.

8. The polymorph modification "C" of atorvastatin L-lysine salt of claim 3, **characterized by** the following X-ray powder-diffraction interlayer distances of the molecule: 18,686; 13,075; 9,177; 7,762; 6,753, 5,250; **4,873;** 4,725; 4,528; 4,211; 3,923; 3,706; 3,455; 3,135 és 3,038 (Å), the characteristic infrared bands are: 3365, 1649, 1596, 1527, 1217, 1031, 851, 877, 810, 747, 582, 505 cm⁻¹, the Raman bands are: 3067, 2914, 1646, 1605, 1529, 1371, 1160, 1001, 826 cm⁻¹.

9. The amorphous modification of atorvastatin L-lysine salt of claim 3, **characterized by** the following characteristic infrared bands: 1648, 1596, 1559, 1530, 1220, 916, 886, 808, 749, 732, 690, 616, 507 cm⁻¹, and Raman bands: 3062, 2922, 1648, 1604, 1530, 1481, 1244, 1159, 999, 820 cm⁻¹.

10. Process for the synthesis of the salts of atorvastatin formed with a basic amino acid of the general formula (I), where the meaning of R is as defined in claim 1, the polymorph modifications thereof, **characterized by** cleaving the protective groups of a protected atorvastatin derivative, preferably a compound of the formula (V) in a solvent, reacting the so obtained atorvastatin acid with a proper amino acid or the salt thereof in a solvent, crystallizing the desired polymorph modification if necessary with the change of solvents.

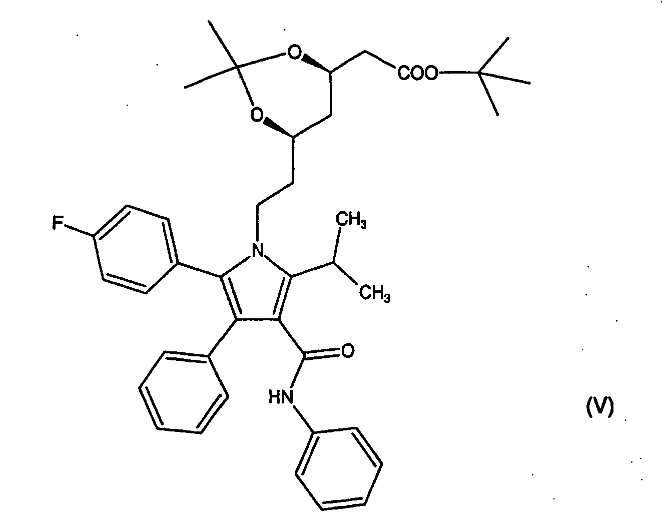

11. Cholesterol level decreasing pharmaceutical composition, **characterized by** having a compound of claim 1-9 as active ingredient in an amount of 0.10-99.90 w/w % and known carriers and auxiliaries in an amount of 0.10-99.90 w/w %.

## Patentansprüche

1. Verbindungen der allgemeinen Formel (I) - wobei die Bedeutung von R eine Gruppe der Formel (II), (III) oder (IV) ist - und die amorphen oder polymorphen Modifikationen davon.

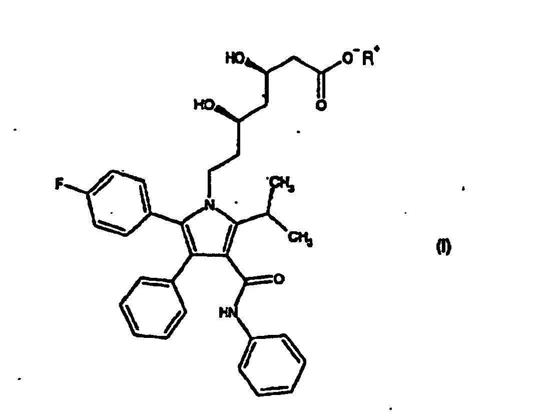

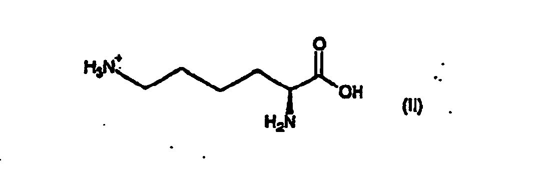

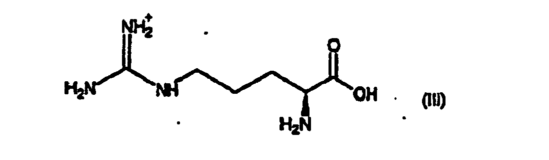

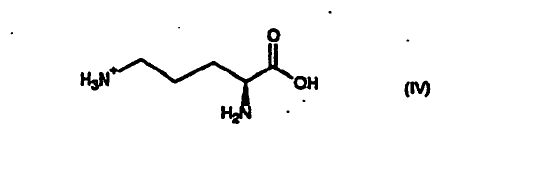

2. Verbindung nach Anspruch 1, bei welcher es sich um Atorvastatin-L-, -D-und -DL-lysinsalze handelt.

3. Verbindung nach Anspruch 1, bei welcher es sich um Atorvastatin-L-lysinsalz handelt.

4. Verbindung nach Anspruch 1, bei welcher es sich um Atorvastatin-L-argininsalz handelt.

5. Verbindung nach Anspruch 1, bei welcher es sich um Atorvastatin-L-omithinsalz handelt.

6. Polymorphe Modifikation "A" von Atorvastatin-L-lysinsalz nach Anspruch 3, **gekennzeichnet durch** die folgenden Schichtabstände in der Röntgenpulverbeugung des Moleküls: 9,351; 5,126; **4,693;** 4,558; 4,239: 4,055; 3,828; 3,617; 3,441; 3,343; 3,203; 3,107; 2,918; 2,709; 2,595(Å), die folgenden charakteristischen Infrarot-Banden sind: 3643, 3322, 1646, 1583, 1423, 1084, 775, 699 cm⁻¹, die folgenden Raman-Banden sind: 2981, 1647, 1480, 734, 677, 635, 513, 200 cm⁻¹.

7. Polymorphe Modifikation "B" von Atorvastatin-L-lysinsalz nach Anspruch 3, **gekennzeichnet durch** die folgenden Schichtabstände in der Röntgenpulverbeugung des Moleküls: 20,971; 13,143; 8,606; 7,512; 6,687; 5,168; 4,778; **4,559;** 4,131; 3,941; 3,839; 3,734; 3,639; 3,236; 3,025; 2,793(Å), die folgenden charakteristischen Infrarot-Banden sind: 3370, 1652, 1412, 1317, 1214, 922, 692, 550 cm⁻¹, die folgenden Raman-Banden sind: 3066, 2950, 1650, 1528, 924, 475, 232, 215 cm⁻¹.

8. Polymorphe Modifikation "C" von Atorvastatin-L-lysinsalz nach Anspruch 3, **gekennzeichnet durch** die folgenden Schichtabstände in der Röntgenpulverbeugung des Moleküls: 18,686; 13,075; 9,177; 7,762; 6,753; 5,250; **4,873** 4,725; 4,528; 4,211; 3,923; 3,706; 3,455; 3,135 und 3,038(Å), die folgenden charakteristischen Infrarot-Banden sind: 3365, 1649, 1596, 1527, 1217, 1031, 851, 877, 810, 747, 582, 505 cm⁻¹, die folgenden Raman-Banden sind: 3067, 2914, 1646, 1605, 1529, 1371, 1160, 1001, 826 cm⁻¹.

9. Amorphe Modifikation von Atorvastatin-L-lysinsalz nach Anspruch 3, **gekennzeichnet durch** die folgenden charakteristischen Infrarot-Banden: 1648, 1596, 1559, 1530, 1220, 916, 886, 808, 749, 732, 690, 616, 507 cm⁻¹ und Raman-Banden: 3062, 2922, 1648, 1604, 1530, 1481, 1244, 1159, 999, 820 cm⁻¹.

10. Verfahren zur Synthese der Atorvastatinsalze, die mit einer basischen Aminosäure der allgemeinen Formel (I), wobei die Bedeutung von R wie in Anspruch 1 definiert ist, gebildet sind, der polymorphen Modifikationen davon, **gekennzeichnet durch** Abspalten der Schutzgruppen eines geschützten Atorvastatinderivats, vorzugsweise einer Verbindung der Formel (V) in einem Lösungsmittel, Umsetzen der so erhaltenen Atorvastatinsäure mit einer angemessenen Aminosäure oder des Salzes davon in einem Lösungsmittel, Kristallisieren der gewünschten polymorphen Modifikation gegebenenfalls unter Austausch von Lösungsmitteln.

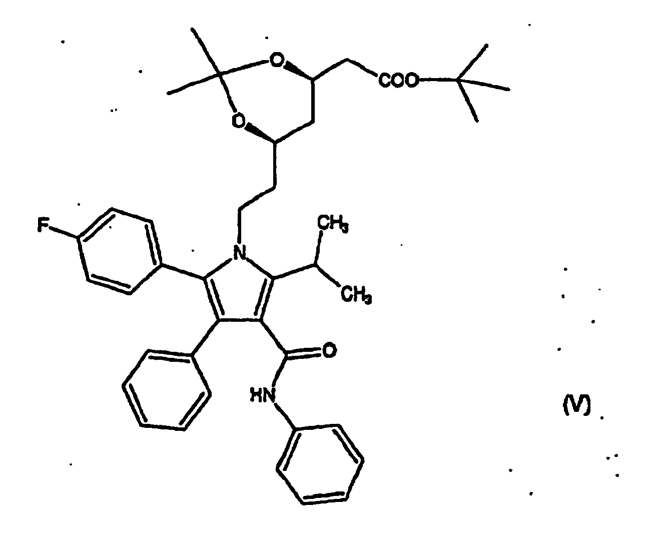

11. Den Cholesterinspiegel senkendes Arzneimittel, **dadurch gekennzeichnet, dass** es als Wirkstoff eine Verbindung nach Anspruch 1-9 in einer Menge von 0,10-99,90 Gew.-% und bekannte Träger und Hilfsstoffe in einer Menge von 0,10-99,90 Gew.-% aufweist.

## Revendications

1. Composés de formule générale (I), dans laquelle R représente un groupe de formule (II), (III) ou (IV), et leurs modifications amorphes ou polymorphes

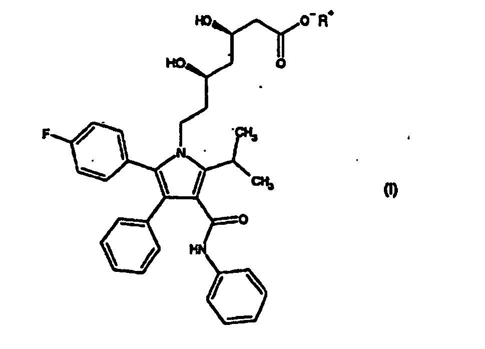

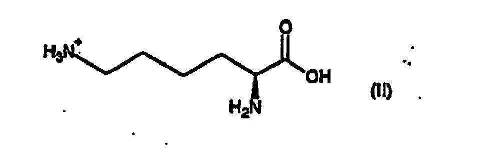

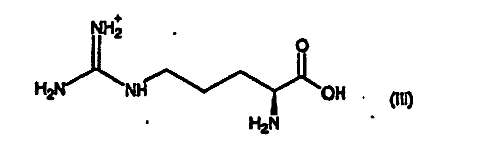

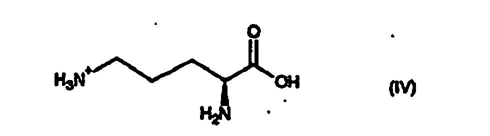

2. Composé selon la revendication 1, qui est les sels de L-, de D- et de DL-lysine de l'atorvastatine.

3. Composé selon la revendication 1, qui est le sel de L-lysine de l'atorvastatine.

4. Composé selon la revendication 1, qui est le sel de L-arginine de l'atorvastatine.

5. Composé selon la revendication 1, qui est le sel de L-ornithine de l'atorvastatine.

6. Modification polymorphe "A" du sel de L-lysine de l'atorvastatine selon la revendication 3, **caractérisée par** les valeurs ci-après des distances entre plans réticulaires de la molécule par diffraction aux rayons X par la méthode des poudres :9,351; 5,126; 4,693; 4,558; 4,239; 4,055; 3,828; 3,617; 3,441; 3,343; 3,203; 3,107; 2,918; 2,709; 2,595(Å), les bandes infrarouges caractéristiques sont 3643, 3322, 1646, 1583, 1423, 1084, 775, 699 cm⁻¹, les bandes Raman sont : 2981, 1647, 1480, 734, 677, 635, 513, 200 cm⁻¹.

7. Modification polymorphe "B" du sel de L-lysine de l'atorvastatine selon la revendication 3, **caractérisée par** les valeurs suivantes des distances entre plans réticulaires de la molécule, obtenues par diffraction aux rayons X par la méthode des poudres : 20,971; 13,143; 8,606; 7,512; 6,687; 5,168; 4,778; 4,559; 4,131; 3,941; 3,839; 3,734; 3,639; 3,236; 3,025; 2,793(Å), les bandes infrarouges caractéristiques sont 3370, 1652, 1412, 1317, 1214, 922, 692, 550 cm⁻¹, les bandes Raman sont : 3066, 2950, 1650, 1528, 924, 475, 232, 215 cm⁻¹.

8. Modification polymorphe "C" du sel de L-lysine de l'atorvastatine selon la revendication 3, **caractérisée par** les valeurs suivantes des distances entre plans réticulaires de la molécule, obtenues par diffraction aux rayons X par la méthode des poudres : 18,686; 13,075; 9,177; 7,762; 6,753; 5,250; 4,873; 4,725; 4,528; 4,211; 3,923; 3,706; 3,455; 3,135 et 3,038(Å), les bandes infrarouges caractéristiques sont 3365, 1649, 1596, 1527, 1217, 1031, 851, 877, 810, 747, 582, 505 cm⁻¹, les bandes Raman sont : 3067, 2914, 1646, 1605, 1529, 1371, 1160, 1001, 826 cm⁻¹.

9. Modification amorphe du sel de L-lysine de l'atorvastatine selon la revendication 3, **caractérisée par** les bandes infrarouges caractéristiques suivantes : 1648, 1596, 1559, 1530, 1220, 916, 886, 808, 749, 732, 690, 616, 507 cm⁻¹, et les bandes Raman : 3062, 2922, 1648, 1604, 1530, 1481, 1244, 1159, 999, 820 cm⁻¹.

10. Procédé de synthèse des sels de l'atorvastatine formés avec un acide aminé basique de formule générale (I) dans laquelle R est tel que défini dans la revendication 1, et de leurs modifications polymorphes, **caractérisé par** le clivage des groupes protecteurs d'un dérivé protégé de l'atorvastatine, de préférence d'un composé de formule (V) dans un solvant, la réaction de l'acide d'atorvastatine ainsi obtenu avec un acide aminé approprié ou un sel de ce dernier dans un solvant, la cristallisation de la modification polymorphe souhaitée, si nécessaire avec changement des solvants.

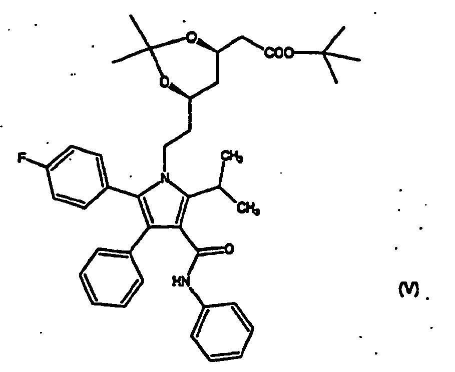

11. Composition pharmaceutique diminuant le taux de cholestérol, **caractérisée en ce qu'**elle contient un composé selon les revendications 1-9 en tant que principe actif en une quantité dé 0,10 à 99,90 p/p, et des excipients et adjuvants connus en une quantité de 0,10 à 99,90 % p/p.
